# EUROPEAN PATENT SPECIFICATION

(11) **EP 2 615 990 B1**
(45) Date of publication and mention of the grant of the patent: **19.08.2020**
(21) Application number: 12801203.6
(22) Date of filing: 27.02.2012
(51) Int. Cl.: A61H 33/00, A61B 18/14, A61B 18/00, A61B 34/20

(54) **IRRIGANT DISTRIBUTION SYSTEM FOR FLEXIBLE ELECTRODES**
SPÜLFLÜSSIGKEITSVERTEILUNGSSYSTEM FÜR FLEXIBLE ELEKTRODEN
SYSTÈME DE DISTRIBUTION IRRIGANT POUR ÉLECTRODES FLEXIBLES

(30) Priority: 16.06.2011 US 201113162417
(43) Date of publication of application: 24.07.2013
(73) Proprietor: St. Jude Medical Atrial Fibrillation Division Inc., St. Paul, Minnesota 55117-9913 (US)
(72) Inventor: CHRISTIAN, Steven, C., New Brighton, MN 55112 (US)
(74) Representative: Kramer Barske Schmidtchen Patentanwälte PartG mbB
(86) International application number: PCT/US2012/026759
(87) International publication number: WO 2012/173673

(56) References cited:
- EP-A1- 2 627 276
- EP-A1- 2 713 924
- US-A- 6 010 500
- US-A1- 2008 091 193
- US-A1- 2008 294 158
- US-A1- 2009 163 911
- US-A1- 2009 163 913
- US-A1- 2009 171 188
- US-A1- 2009 171 188
- US-A1- 2010 137 859
- US-A1- 2010 174 177
- US-A1- 2010 174 177
- US-A1- 2010 286 684
- US-A1- 2011 092 969
- US-B1- 6 616 655
- US-B1- 7 389 148
- US-B2- 7 776 034
- US-B2- 7 815 635
- US-B2- 7 826 905

## Description

### BACKGROUND OF THE INVENTION

### a. Field of the Invention

The instant disclosure relates generally to ablation electrode assemblies including a flexible electrode shell and an irrigant distribution element.

### b. Background Art

Electrophysiology catheters are used in a variety of diagnostic and/or therapeutic medical procedures to diagnose and/or correct conditions such as atrial arrhythmias, including for example, ectopic atrial tachycardia, atrial fibrillation, and atrial flutter. Arrhythmias can create a variety of conditions including irregular heart rates, loss of synchronous atrioventricular contractions and stasis of blood flow in a chamber of a heart which can lead to a variety of symptomatic and asymptomatic ailments and even death.

A medical procedure in which an electrophysiology catheter is used includes a first diagnostic catheter deployed through a patient's vasculature to a patient's heart or a chamber or vein thereof. An electrophysiology catheter that carries one or more electrodes can be used for cardiac mapping or diagnosis, ablation and/or other therapy delivery modes, or both. Once at the intended site, treatment can include, for example, radio frequency (RF) ablation, cryoablation, laser ablation, chemical ablation, high-intensity focused ultrasound-based ablation, microwave ablation. An electrophysiology catheter imparts ablative energy to cardiac tissue to create one or more lesions in the cardiac tissue and oftentimes a contiguous or linear and transmural lesion. This lesion disrupts undesirable cardiac activation pathways and thereby limits, corrals, or prevents errant conduction signals that can form the basis for arrhythmias.

During RF ablation, local temperature elevation can result in coagulum formation on the ablation electrode, resulting in an impedance rise. As the impedance increases, more energy is passed through the portion of the electrode without coagulation, creating even higher local temperatures and further increasing coagulum formation and the impedance. Finally, enough blood coagulates onto the electrode that no energy passes into the targeted tissue, thereby requiring the catheter to be removed from the vascular system, the electrode to be cleaned, and the catheter to be repositioned within the cardiac system at the desired location. Not only can this process be time consuming, but it can be difficult to return to the previous location because of the reduced electrical activity in the targeted tissue, which has been previously ablated. Recent studies have also demonstrated the formation of a so-called soft thrombus in RF ablation. The formation of the soft thrombus results from heat induced protein denaturation and aggregation and occurs independently of heparin concentration in serum. In addition, RF ablation can generate significant heat, which, if not controlled, can result in excessive tissue damage, such as tissue charring, steam pop, and the like.

Accordingly, it can be desirable to monitor and/or control the temperature of ablation electrode assemblies and/or targeted tissue. Thermal sensors such as thermocouples and/or thermistors may be used to monitor the temperature of ablation electrode assemblies. RF ablation catheters can be configured to provide temperature feedback during RF ablation via the thermal sensors in order to adjust one or more parameters of an RF ablation cycle. Accordingly, it can be desirable to improve temperature correlation between the electrode and tissue interface in order to provide more accurate temperature feedback with respect to the tissue temperature for controlling energy delivery and/or other parameters during an RF ablation cycle. It can also be desirable to use ablation electrode assemblies to provide irrigation fluid during RF ablation. In addition, it can be desirable to provide a flexible electrode that may be better configured to conform to the tissue surface targeted for treatment by deflecting and/or undergoing deformation when the flexible electrode comes into physical contact with the targeted tissue. In this way, a flexible electrode can better accommodate cardiac anatomy, and conformation of the electrode with the cardiac anatomy can ensure more efficient energy delivery.

US 2009/163913 A1 relates to an irrigated ablation catheter assembly comprising a catheter including a catheter shaft having a lumen, an irrigated ablation electrode assembly including a proximal portion and a distal portion disposed distally of the proximal portion, the proximal portion having a body portion including an outer surface, an inner cavity within the body portion, and at least one passageway that extends from the inner cavity to the outer surface of the body portion, and a distal portion having a distal end, and a flow member having a body including a proximal end and a distal end, wherein the proximal end of the flow member is coupled or connected to the catheter shaft and the distal end of the flow member is disposed about the proximal portion of the electrode assembly.

US 2009/171188 A1 and US 7,389,148 B1 both relate to flexible electrodes for ablation purposes.

US 2010/137859 A1 relates to an irrigated ablation catheter comprising a shaft having a proximal end, a distal end, and at least one irrigation lumen therein, an ablation electrode, and a manifold disposed between and coupled to each of said distal end of said shaft and said electrode, at least a portion of said manifold constructed of a flexible material configured to allow said manifold and said electrode to be deflected, said manifold including a body defined by an outer surface, an inner cavity disposed within said body and configured for fluid communication with said irrigation lumen, and an irrigation port disposed within said body extending from said inner cavity to said outer surface of said body and configured to allow for an irrigating fluid to be distributed from said manifold.

US 2010/286684 A1 relates to an irrigated catheter ablation apparatus comprising an elongated body having a distal end, a proximal end, and at least one fluid lumen extending longitudinally therein, a plurality of segmented ablation electrodes on a distal portion of the elongated body, the plurality of segmented ablation electrodes being spaced from the proximal end and from the distal end of the elongated body by electrically nonconductive segments, the plurality of segmented ablation electrodes being spaced from each other longitudinally by electrically nonconductive segments, such that for each segmented ablation electrode that is longitudinally disposed next to one of the electrically nonconductive segments, an edge is formed between an electrode end of the segmented ablation electrode and a nonconductive segment end of the electrically nonconductive segment, a plurality of elution holes being disposed adjacent to the edges which are between the electrode ends of the segmented ablation electrodes and the nonconductive segment ends of the electrically nonconductive segments, and a plurality of ducts establishing fluid communication between the elution holes and the at least one fluid lumen.

### BRIEF SUMMARY OF THE INVENTION

It is desirable to have improved temperature correlation between the electrode of the ablation electrode assembly and the tissue interface. It is also desirable, in some embodiments, to include a mechanism to irrigate the ablation electrode assemblies and/or targeted areas in a patient's body with biocompatible fluids, such as saline solution, in order to reduce charring and inhibit the formation of coagulum and/or soft thrombus, as well as to enable deeper and/or greater volume lesions as compared to conventional, non-irrigated catheters at identical power settings. This can, in turn, enable greater energy delivery during RF ablation. The flow of biocompatible fluids (*i.e.,* irrigation fluids) can be turbulent in order to provide an enveloping flow pattern adjacent to the surface of the ablation electrode assemblies for mixing with, displacing, and/or diluting blood that can be in contact with the ablation electrode assemblies in order to prevent stasis and the formation of coagulum. In addition, it may be desirable for the electrode to conform to cardiac anatomy in order to improve energy efficiency during RF ablation.

An ablation electrode assembly in accordance with an embodiment of the disclosure has a longitudinal axis, an electrode core member, an electrode shell, and an irrigant distribution element. The electrode core member comprises a thermal insulator having a reduced thermal conductivity. The electrode core member has a distal end; a proximal end; and at least one irrigation passageway. The electrode core member further comprises an outer surface and an inner surface defining a cavity. The at least one irrigation passageway extends from the inner cavity to the outer surface of the electrode core member. The electrode core member further comprises an axially extending passageway extending from the inner cavity of the electrode core member toward the distal end of the electrode shell. The ablation electrode assembly further includes at least one port extending from the axially extending passageway to the distal end of the electrode shell. The port is oriented at an acute angle relative to the longitudinal axis of the ablation electrode assembly in accordance with an embodiment of the disclosure. At least a portion of the circumference and at least a portion of the length of the axially extending passageway can include a coating of an electrically non-conductive material.

The electrode shell comprises an electrically conductive material. The electrode shell defines an inner volume and has a distal end; and a proximal end. The proximal end of the electrode shell is configured for connection to the distal end of the electrode core member. The electrode shell is sufficiently flexible for deflection of the distal end of the electrode shell relative to the longitudinal axis of the ablation electrode assembly. The irrigant distribution element has a proximal end; and a distal end. The distal end of the irrigant distribution element defines a circumferential irrigation port between the irrigant distribution element and the electrode core member. In accordance with a first embodiment of the disclosure, at least a portion of the electrode shell includes a first set of projections defining at least in part a corresponding first set of recesses, and at least a portion of the electrode shell includes a second set of projections defining at least in part a corresponding second set of recesses. At least one of the first set of projections is configured to interlock with at least one of the second set of recesses, and at least one of the second set of projections is configured to interlock with at least one of the first set of recesses. Each of the first set of projections and each of the second set of projections can be trapezoidal in shape in an embodiment of the disclosure. Each of the first set of projections and each of the second set of projections can be rounded in shape in an embodiment of the disclosure.

In accordance with a second embodiment of the disclosure, the electrode shell can comprise wound or braided metallic wires. In accordance with a third embodiment of the disclosure, the electrode shell can comprise a polymer (for example and without limitation, silicone) having electrically conductive particles dispersed therein at a predefined density to achieve a desired electrical conductivity. The particles can comprise gold, silver, platinum, iridium, titanium, tungsten, or a combination thereof in accordance with various embodiments of the invention.

The irrigant distribution element can comprise an annular ring and can further comprise a fluid shaping member, such as a channel, rifling, boss, hump, chamfer, or combination thereof, in order to improve fluid flow characteristics of the irrigation fluid.

### BRIEF DESCRIPTION OF THE DRAWINGS

FIG. 1 is a diagrammatic view of a system for performing one more diagnostic and/or therapeutic functions in association with cardiac tissue.
FIG. 2 is an isometric partially transparent view of an ablation electrode assembly in accordance with a first embodiment of the disclosure.
FIG. 3 is a cross-sectional view of the ablation electrode assembly of FIG. 2.
FIG. 4 is an isometric partially transparent view of the ablation electrode assembly of FIG. 2 illustrating the flexibility of the outer shell of the ablation electrode assembly of FIG. 2.
FIG. 5A is an isometric view of a portion of the outer shell of the ablation electrode assembly of FIG. 2 in accordance with a first embodiment of the invention.
FIG. 5B is an isometric view of a portion of the outer shell of the ablation electrode assembly of FIG. 2 in accordance with a second embodiment of the invention.
FIG. 6 is an isometric partially transparent view of an ablation electrode assembly in accordance with a second embodiment of the disclosure and in accordance with the present invention.
FIG. 7 is a cross-sectional view of the ablation electrode assembly of FIG. 6.
FIG. 8 is an isometric partially transparent view of an ablation electrode assembly in accordance with a third embodiment of the disclosure.
FIG. 9 is an isometric partially transparent view of an ablation electrode assembly in accordance with a fourth embodiment of the disclosure.

### DETAILED DESCRIPTION OF THE DISCLOSURE

The instant disclosure generally relates to irrigated ablation electrode assemblies. For purposes of this description, similar aspects among the various embodiments described herein will be referred to by similar reference numbers. As will be appreciated, however, the structure of the various aspects can be different among the various embodiments.

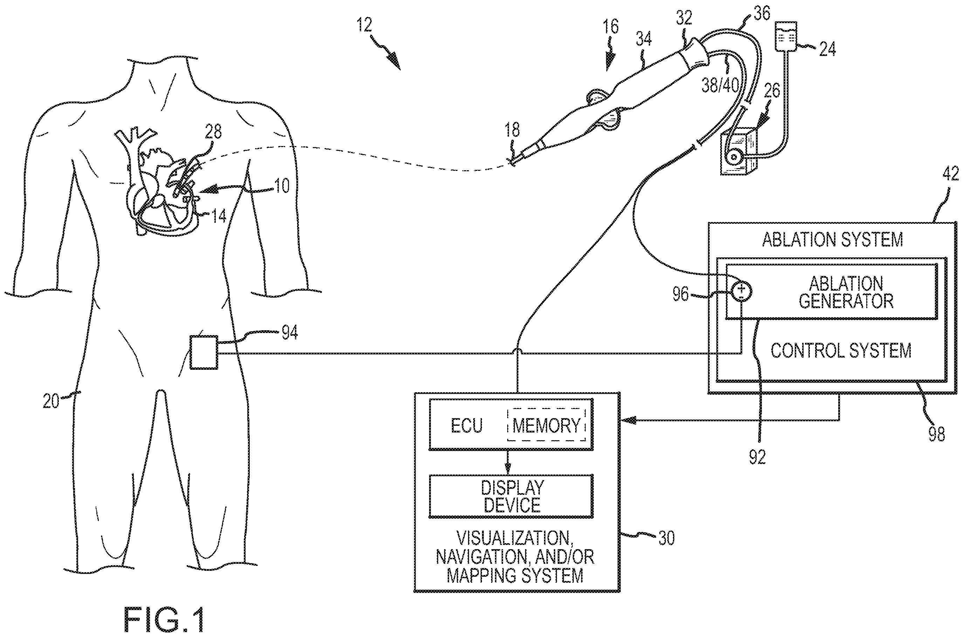

Referring to FIG. 1, an ablation electrode assembly 10 can comprise part of an irrigated catheter system 12 for examination, diagnosis, and/or treatment of internal body tissues (e.g., targeted tissue areas 14). In an exemplary embodiment, the irrigated catheter assembly can comprise an ablation catheter 16 (e.g., radio frequency (RF), cryoablation, ultrasound, etc.). The instant disclosure generally refers to RF ablation electrodes and electrode assemblies, but it is contemplated that the instant disclosure is equally applicable to any number of other ablation electrodes and electrode assemblies where the temperature of the device and of the targeted tissue areas can be factors during diagnostic and/or therapeutic medical procedures.

Still referring to FIG. 1, the irrigated catheter assembly includes a catheter shaft 18 that is an elongate, tubular, flexible member configured for movement within a body. The catheter shaft 18 can be introduced into a blood vessel or other structure within a body 20 through a conventional introducer. The catheter shaft 18 can be steered or guided through a body to a desired location such as targeted tissue areas 14 with pullwires, tension elements, so-called push elements, or other means known in the art.

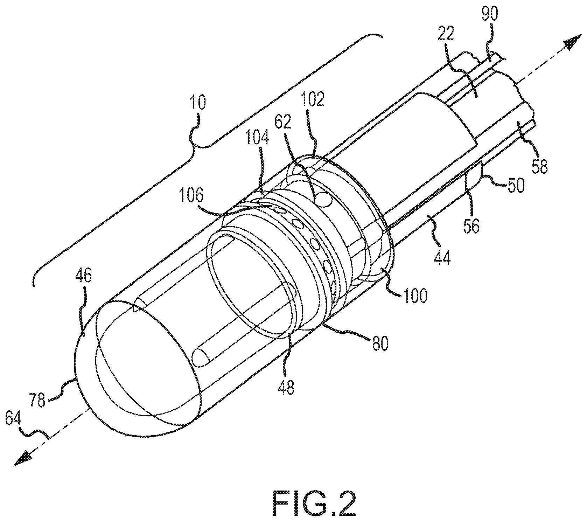

The irrigated catheter assembly further includes at least one fluid lumen or fluid delivery tube 22 disposed within the catheter shaft 18, best shown in FIG. 2. The fluid delivery tube 22 is configured to supply fluid to the ablation electrode assembly 10. Referring now to FIGS. 1-2, the fluid delivery tube 22 of the irrigated catheter assembly can be connected to a fluid source 24 providing a biocompatible fluid such as saline, or a medicament, through a pump 26, which can comprise, for example, a fixed rate roller pump or variable volume syringe pump with a gravity feed supply from the fluid source for irrigation. The fluid source 24 and/or pump 26 is conventional in the art. The fluid source 24 and/or pump 26 can comprise a commercially available unit sold under the name Cool Point™, available from St. Jude Medical, Inc. in an embodiment.

Referring now to FIG. 1, the irrigated catheter assembly can further include one or more positioning electrodes 28 mounted in or on the catheter shaft 18. The electrodes 28 can comprise, for example, ring electrodes. The electrodes 28 can be used, for example, with a visualization, navigation, and mapping system 30. The electrodes 28 can be configured to provide a signal indicative of both a position and orientation of at least a portion of the catheter shaft 18. The visualization, navigation, and/or mapping system 30 with which the electrodes 28 can be used can comprise an electric field-based system, or, sometimes referred to as an impedance based system, such as, for example, that having the model name ENSITE NAVX (aka EnSite Classic as well as newer versions of the EnSite system, denoted as ENSITE VELOCITY) and commercially available from St. Jude Medical, Inc. and as generally shown with reference to U.S. Patent No. 7,263,397 titled "Method and Apparatus for Catheter Navigation and Location and Mapping in the Heart,". The visualization, navigation, and/or mapping system 30 can include an electronic control unit (ECU) and display device. The ECU can comprise a programmable microprocessor or microcontroller, but can alternatively comprise an application specific integrated circuit (ASIC). The ECU can include a central processing unit (CPU) and an input/output (I/O) interface through which the ECU can receive input data and can generate output data. The ECU can also have a memory, and the input data and/or output data acquired and generated by the ECU can be stored in the memory of the ECU.

In accordance with an electric field-based system, the electrodes 28 can be configured to be responsive to an electric field transmitted within the body 20 of the patient. The electrodes 28 can be used to sense an impedance at a particular location and transmit a representative signal to an external computer or processor. In other exemplary embodiments, however, the visualization, navigation, and/or mapping system 30 can comprise other types of systems, such as, for example and without limitation: a magnetic field-based system such as the CARTO System (now in a hybrid form with impedance- and magnetically-driven electrodes) available from Biosense Webster, and as generally shown with reference to one or more of U.S. Patent Nos. 6,498,944 entitled "Intrabody Measurement," 6,788,967 entitled "Medical Diagnosis, Treatment and Imaging Systems," and 6,690,963 entitled "System and Method for Determining the Location and Orientation of an Invasive Medical Instrument," or the gMPS system from MediGuide Ltd. of Haifa, Israel (now owned by St. Jude Medical, Inc.), and as generally shown with reference to one or more of U.S. Patent Nos. 6,233,476 entitled "Medical Positioning System," 7,197,354 entitled "System for Determining the Position and Orientation of a Catheter," and 7,386,339 entitled "Medical Imaging and Navigation System." In accordance with a magnetic field-based system, the catheter can be configured to include field sensors (*e.g.,* coils) responsive to a magnetic field transmitted through the body 20 of the patient to sense the strength of the field at a particular location and transmit a representative signal to an external computer or processor. Such field sensors can comprise one or more metallic coils located on or within the catheter shaft 18 in a magnetic field-based system. As noted above, a combination electric field-based and magnetic field-based system such as the CARTO 3 System also available from Biosense Webster, and as generally shown with reference to U.S. Patent No. 7,536,218 entitled "Hybrid Magnetic-Based and Impedance-Based Position Sensing," can be used. In accordance with a combination electric field-based and magnetic field-based system, the catheter can include both electrodes 28 as impedance-based electrodes and one or more magnetic field sensing coils. Commonly available fluoroscopic, computed tomography (CT), and magnetic resonance imaging (MRI)-based systems can also be used.

The irrigated catheter assembly can include other conventional components such as, for example and without limitation, conductors associated with the electrodes, and possibly additional electronics used for signal processing, visualization, localization, and/or conditioning. The irrigated catheter assembly can further include multiple lumens for receiving additional components. Still referring to FIG. 1, the irrigated catheter assembly can further include a cable connector or interface 32 and a handle 34. The cable connector or interface 32 can provide mechanical, fluid, and electrical connection(s) for cables 36, 38, 40 extending from the pump 26 and/or an ablation system 42 as described in more detail below. The cable connector or interface 32 can be conventional in the art and can be disposed at the proximal end of the irrigated catheter assembly. The handle 34 can provide a location for the clinician to hold the irrigated catheter assembly and can further provide means for steering or guiding the catheter shaft 18 within the body 20 as known in the art. Catheter handles are generally conventional in the art and it will be understood that the construction of the handle can vary. In an embodiment, for the purpose of steering the catheter shaft 18 within the body 20, the handle 34 can be substituted by a controllable robotic actuator.

Referring now to FIGS. 1-4, ablation electrode assembly 10 can be connected to and/or coupled with the catheter shaft 18. Ablation electrode assembly 10 can be disposed at or near the distal end of the catheter shaft 18. Ablation electrode assembly 10 can be disposed at the extreme distal end (*e.g.,* tip) of the catheter shaft 18. Referring now to FIGS. 2-3, the ablation electrode assembly 10 can include an electrode core member 44 and an electrode shell 46 in accordance with a first embodiment of the disclosure. The lengths and/or diameters of ablation electrode assembly 10, electrode core member 44, electrode shell 46, as well as portions thereof, can vary depending on the design of ablation electrode assembly 10. The electrode shell 46 can be about four millimeters in length in an embodiment. Although four millimeters is mentioned in detail, the length of the electrode shell 46 can vary in accordance with various embodiments of the invention.

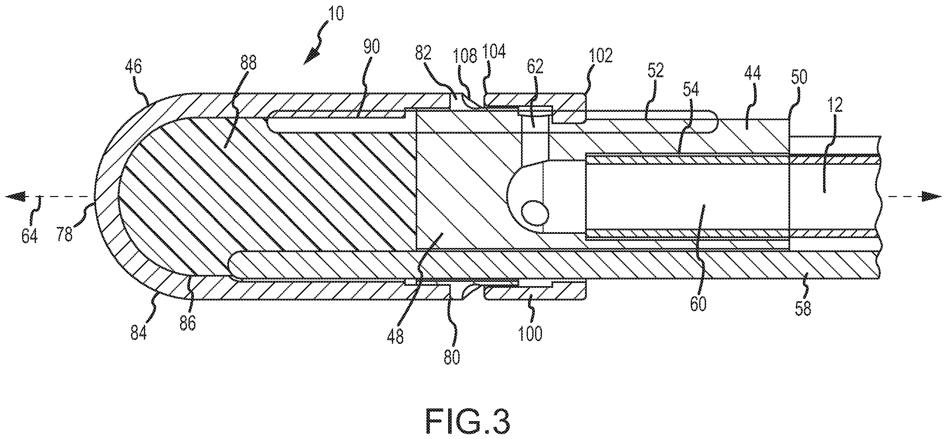

Electrode core member 44 is configured for coupling the ablation electrode assembly 10 to the catheter shaft 18 and for routing various components to the electrode shell 46. Electrode core member 44 has a first end 48 and a second end 50. First end 48 can be a distal end, and second end 50 can be a proximal end in accordance with an embodiment of the disclosure. Electrode core member 44 can be generally cylindrical in shape. The first end 48 of the electrode core member 44 can be generally flat in accordance with an embodiment of the disclosure. The second end 48 of the electrode core member 44 can be partially spherical or generally hemispherical in shape in accordance with other embodiments of the disclosure. Although these particular shapes are mentioned in detail, the shape of the first end 48 of the electrode core member 44 can vary in accordance with various embodiments of the disclosure. The second end 50 of the electrode core member 44 can be configured for coupling and/or connecting electrode core member 44 with the catheter shaft 18. The second end 50 of the electrode core member 44 can also be configured to receive the fluid delivery tube 22. The electrode core member 44 can include multiple lumens for receiving any number of components (e.g., wires and the like) which can be routed through the electrode core member 44. As best illustrated in FIG. 3, the electrode core member 44 also has an outer surface 52 and an inner surface 54. Referring back to FIG. 2, the outer surface 52 of the electrode core member 44 can include at least one channel 56 for receiving a thermal sensor 58.

Accordingly, the ablation electrode assembly 10 can include at least one thermal sensor 58 in accordance with an embodiment of the disclosure as best shown in FIGS. 2-3. The ablation electrode assembly 10 can include three thermal sensors 58 in accordance with an embodiment of the disclosure. The thermal sensors 58 can be substantially equally spaced around the periphery or circumference of the electrode core member 44. Although three sensors that are substantially equally spaced are mentioned in detail, the ablation electrode assembly 10 can include fewer or more thermal sensors 58 in other embodiments and the location of the thermal sensors 58 can vary in other embodiments. For example, in an embodiment, a single thermal sensor 58 may be centered within the ablation electrode assembly 10. Thermal sensors 58 can be connected and/or coupled to electrode core member 44 (and/or ablation electrode assembly 10) in any manner that is conventional in the art to hold thermal sensors 58 in place relative to electrode core member 44 (and/or ablation electrode assembly 10). Thermal sensors 58 are configured for measurement and temperature control/regulation of ablation electrode assembly 10. Thermal sensors 58 can be any mechanism known to one of ordinary skill in the art, including for example and without limitation, thermocouples and/or thermistors. Thermal sensors 58 can comprise other types of devices, such as for example and without limitation, devices for determining pressure, temperature and a flow parameter of a flowing fluid available from Radi Medical Systems AB, and as generally shown with reference to at least U.S. Patent No. RE39,863 entitled "Combined flow, pressure and temperature sensor."

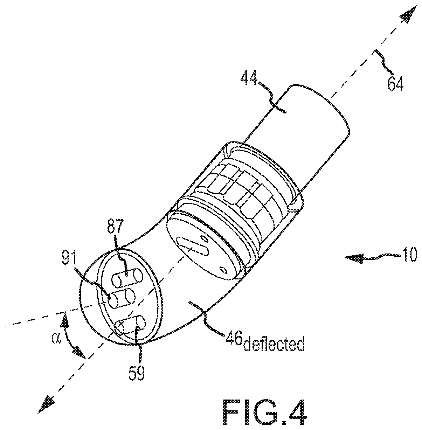

At least a portion of the thermal sensors 58 can also be routed through the electrode shell 46. At least a portion of the thermal sensors 58 can be surface mounted to an inner surface 86 of the electrode shell 46 in accordance with an embodiment of the disclosure. At least a portion of the thermal sensors 58 can be surface mounted to the inner surface 86 of the electrode shell 46 in any manner known to those of ordinary skill in the art. Referring now to FIG. 4, the electrode shell 46 can include a receptacle 59 for receiving at least a portion of the thermal sensor 58 described hereinabove which can be routed through the electrode shell 46 in accordance with an embodiment of the disclosure. For example and without limitation, the electrode shell 46 can include a tab extension (not shown) extending radially inwardly from the inner surface 86 of the electrode shell 46 having at least one receptacle through which at least a portion of the thermal sensor 58 can be routed. Although a tab extension is mentioned in detail, other structures can be utilized to provide a receptacle through which the thermal sensors 58, or any number of other components, can be routed.

Inner surface 54 of the electrode core member 44 defines an inner cavity 60 as best illustrated in FIG. 3. In an embodiment of the disclosure, the electrode core member 44 includes an irrigation passageway 62 that extends from the inner cavity 60 to the outer surface 52 of the electrode core member 44. Electrode core member 44 includes a plurality of irrigation passageways 62 in an embodiment. Each of the irrigation passageways 62 extend from the inner cavity 60 of the electrode core member 44 to the outer surface 62 of the electrode core member 44. Each of the irrigation passageways 62 can be located closer to the first end 48 of the electrode core member 44 than to the second end 50 of the electrode core member 44 in accordance with an embodiment of the disclosure. Each of the irrigation passageways 62 can generally extend radially outwardly. The ablation electrode assembly 10 can include a longitudinal axis 64. In an embodiment, each of the irrigation passageways 62 can be oriented at about 90 degrees relative to the longitudinal axis 64 of the ablation electrode assembly 10. In accordance with other embodiments, one or more of the irrigation passageways 62 can be angled generally toward the first end 48 of the electrode core member 44 at an acute angle (e.g., between about 20 to about 70 degrees, and for some embodiments, between about 30 to about 65 degrees) with respect to the longitudinal axis 64 of the ablation electrode assembly 10. The orientation of the irrigation passageways 62 vary depending on the design of the ablation electrode assembly 10. The irrigation passageways 62 of the electrode core member 44 can be straight or curved in various embodiments of the disclosure. In accordance with an embodiment of the disclosure, the irrigation passageways 62 of the electrode core member 44 can be diametrically opposed to each other around the perimeter or circumference of the electrode core member 44. Each of the irrigation passageways 62 can be generally tubular and can have a constant diameter along their length. In an embodiment, each of the irrigation passageways 62 can have a diameter ranging in size from about 0.008 inches (about 0.20 millimeters or about 1.04F) to about 0.015 inches (about 0.38 millimeters or about 1.95F), and for some embodiments between about 0.010 inches (about 0.25 millimeters or about 1.30F) to about 0.012 inches (about 0.30 millimeters or about 1.56F). Alternate configurations having various shapes and diameters, for example, along all or portions of the length of the irrigation passageways 62 can be used in various embodiments. Each of the irrigation passageways 62 can be configured to provide proximal delivery of irrigation fluid. Delivery of irrigation fluid generally reduces char, thrombus formation, and coagulum formation, thereby enabling greater energy delivery during RF ablation.

Electrode core member 44 can comprise a thermal insulator having a reduced thermal conductivity. Electrode core member 44 can be thermally nonconductive in accordance with an embodiment of the disclosure. Electrode core member 44 can comprise an electrically nonconductive material in accordance with an embodiment of the disclosure. In general, the electrode core member 44 is lower in thermal conductivity, and preferably substantially lower, than the electrode shell 46. Electrode core member 44 can comprise a reduced thermally conductive polymer in accordance with an embodiment of the disclosure. A reduced thermally conductive polymer is one with physical attributes that decrease heat transfer by about 10% or more, provided that the remaining structural components are selected with the appropriate characteristics and sensitivities desired for the ablation electrode assembly 10. One reduced thermally conductive material can include polyether ether ketone (PEEK). Additional examples of thermally nonconductive or reduced thermally conductive materials that can be useful in conjunction with the instant disclosure include, but are not limited to, high density polyethylene (HDPE), polyimide thermoplastic resins, such as those resins sold under the trademark ULTEM® and as generally available from General Electric Plastics (now known as SABIC Innovative Plastics), polyaryletherketones, polyurethane, polypropylene, oriented polypropylene, polyethylene, crystallized polyethylene terephthalate, polyethylene terephthalate, polyester, polyetherimide, acetyl, ceramics, and/or various combinations thereof. Electrode core member 44 can also comprise other plastic materials such as silicone or polyether block amides such as those sold under the trademark PEBAX® and generally available from Arkema France in other embodiments of the disclosure.

Electrode shell 46 is a relatively thin shell defining an inner volume as best illustrated in FIGS. 2-3. Electrode shell 46 is configured to improve temperature correlation between the electrode and tissue interface because it is a relatively thin shell in place of a solid mass *(i.e.,* requiring less time for the electrode shell 46 to register an increased temperature due to the application of energy). Electrode shell 46 can be a relatively thin shell *(i.e.,* have a small thickness) and can be external to and/or surround at least the first end 48 of the electrode core member 44. Electrode shell 46 can comprise a single layer in accordance with an embodiment of the disclosure.

At least a portion of electrode shell 46 may be generally flexible in an embodiment. For example, at least a portion of electrode shell 46 may be configured to conform to the targeted tissue 14, and may therefore, deflect and/or undergo deformation when electrode shell 46 comes into physical contact with the targeted tissue 14. In particular, the electrode shell 46 can be sufficiently flexible so that at least a distal portion of electrode shell 46 may be configured for deformation and/or deflection in a number of directions relative to the longitudinal axis 64 of ablation electrode assembly 10.

Referring now to FIG. 4, the electrode shell 46 is shown in a deflected and/or deformed position 46_{deflected}, and is schematically shown deflected at an angle α relative to axis 64. Although this particular deflection is illustrated, electrode shell 46 may be deflected and/or deformed in various other ways, including in a direction along different axes other than the axis of the ablation electrode assembly 10. Deflection and/or deformation of the electrode shell 46 can allow the electrode shell 46 to conform to cardiac anatomy in order to improve energy efficiency during RF ablation.

Electrode shell 46 can be comprised of any electrically, and potentially thermally, conductive material known to those of ordinary skill in the art for the delivery of ablative energy to targeted tissue areas. Examples of electrically conductive materials include gold, platinum, iridium, palladium, stainless steel, and/or any combination thereof. In particular, a combination of platinum and iridium can be used in various combinations. Electrode shell 46 can be fabricated or constructed in accordance with any method or technique known to one of ordinary skill in the art. For example and without limitation, electrode shell 46 can be fabricated or constructed using so-called deep drawn metal forming techniques, metal-punching techniques, electro forming techniques (e.g., electroforming over a sacrificial form that can include rods or other internal forms that melt or are subsequently dissolved), powdered metal techniques (e.g., pressing powered metal into a slug, sintering at high heat, and then covering the pressed and sintered slug with a metallic covering member), liquid metal injection molding (MIM) techniques, and the like. The powered metal techniques can also include sacrificial members, and the pressed and sintered slug can itself conduct fluid and thermal energy inside, around, and against the metallic covering.

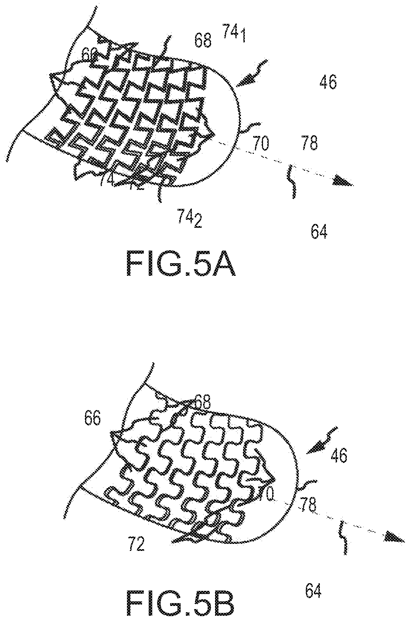

Referring to FIGS. 5A-5B, in accordance with an embodiment of the present invention wherein the electrode shell 46 comprises a metal, the electrode shell 46 is comprised of a single member that is formed into a helix, or spiral, and extends from distal end 78 to proximal end 80 or at least a portion thereof. For example and without limitation, at least a portion of the electrode shell 46 can be similar to the tip element described and illustrated in U.S. Patent Application Publication No. 2010/0174177 titled "Magnetically Guided Catheter." Referring again to FIGS. 5A-5B, at least a portion of the electrode shell 46 includes a first set of projections 66 defining at least in part a corresponding first set of recesses 68. At least a portion of the electrode shell 46 includes a second set of projections 70 defining at least in part a corresponding second set of recesses 72. The first set of projections 66 and the second set of projections 70 are alternately spaced and extend away from the electrode shell 46 in opposite directions from one another along the length of the helix or spiral. In particular, each of the first set of projections 66 extend proximally *(i.e.,* away from the distal end 78 of the electrode shell 46), and each of the second set of projections 70 extend distally *(i.e.,* toward the distal end 78 of the electrode shell 46). The first set of projections 66 can be staggered and/or offset from the second set of projections 70 such that the first set of projections are positioned between the second set of projections 70. The first set of recesses 68 and the second set of recesses 72 are complementary in shape to an outer contour of the first set of projections 66 and the second set of projections 70, respectively, but inversely shaped from same. In the embodiment of the disclosure illustrated in FIG. 5A, each of the first set of projections 66, the first set of recesses 68, the second set of projections 70, and the second set of recesses 72 are trapezoidal in shape. Although a trapezoidal shape is mentioned in detail, the projections 66, 70 and recesses 68, 72 can be other any number of other shapes in accordance with other embodiments of the disclosure. For example and without limitation, in the embodiment of the disclosure illustrated in FIG. 5B, each of the first set of projections 66, the first set of recesses 68, the second set of projections 70, and the second set of recesses 72 can be rounded (e.g., teardrop) in shape.

The electrode shell 46 can be fabricated such that the projections 66 from a section of the electrode shell 46 extend into, and are captured within, recesses 72 from an adjacent section of electrode shell 46 to form an interlocking arrangement. In addition, projections 70 from a section of the electrode shell 46 extend into, and are captured within, recesses 68 from an adjacent section of electrode shell 46 to form an interlocking arrangement. Accordingly, at least one of the first set of projections 66 is configured to interlock with at least one of the second set of recesses 72, and at least one of the second set of projections 70 is configured to interlock with at least one of the first set of recesses 68. Due to projections 66, 70 being complementary in shape to recesses 72, 68, respectively, and thus defining sockets or compartments for projections 66, 70, projections 66, 70 are moveable only a defined distance within recesses 72, 68. In particular, electrode shell 46 is positionable to create a space or gap 74 between leading edges of projections 66, 70 and inner edges or recesses 72, 68, respectively. Projections 66, 70 and recesses 68, 72 of the electrode shell 46 extend along at least more than half the length of electrode shell 46. For example and without limitation, projections 66, 70 and recesses 68, 72 extend along at least two thirds of the length of the electrode shell 46. Although these lengths are mentioned in detail, projections 66, 70 and recesses 68, 72 can extend for more or less of the entire length of the electrode shell 46 in accordance with various embodiments of the disclosure. For example and without limitation, the projections 66, 70 and recesses 68, 72 can be uniformly spaced along the length of the electrode shell 46 and can also be uniformly spaced around the perimeter (e.g., circumference) of the electrode shell 46. Although uniform spacing is mentioned in detail, projections 66, 70 and recesses 68, 72 can be differently spaced along the length and/or perimeter of the electrode shell 46 in accordance with various embodiments of the disclosure. For example and without limitation, the projections 66, 70 and recesses 68, 72 can be uniformly sized along the length of the electrode shell 46 and can be uniformly sized around the perimeter (e.g., circumference) of the electrode shell 46. Although uniform sizing is mentioned in detail, projections 66, 70 and recesses 68, 72 can be differently sized along the length and/or perimeter of the electrode shell 46 in accordance with various embodiments of the disclosure.

As a consequence of gaps 74, and also the complementary shape of projections 66, 70 and recesses 68, 72, projections 66, 70 are provided a freedom of movement within recesses 68, 72 without being able to be removed therefrom. Accordingly, sections of electrode shell 46 can move toward and away from each other a defined distance to decrease and increase, respectively, gaps 74. The ability of sections of electrode shell 46 to move toward and away from each other a defined distance to decrease and increase, respectively, gaps 74 can constrain the flexure of the electrode shell 46 and limit the range of extensibility of the electrode shell 46.

It is possible for sections of electrode shell 46 to move relative to one another in multiple ways. For example, the electrode shell 46 can be compressed so that all of gaps 74 are closed, or nearly closed, to reduce the longitudinal length of the electrode shell 46 by the cumulative dimensions of gaps 74 along a longitudinal axis 64. Additionally, sections of electrode shell 46 can exhibit cascaded and/or sequential movement along longitudinal axis 64 wherein some gaps 74 are closed along longitudinal axis 64 while other gaps remain open, either partially or fully. This allows gaps 74 between any adjacent sections of the electrode shell 46 to be opened or closed in an uneven or non-uniform manner. As such, gaps 74₁ on a first portion of the perimeter (e.g., circumference) of the electrode shell 46 may be closed while gaps 74₂ on another second portion (e.g., opposing the first portion) of the electrode shell may be opened. The result of such a configuration is that the electrode shell 46 curves in the direction of the closed gaps 74₁ and away from the direction of the opened gaps 74₂. It can be appreciated that movement in vertical and horizontal planes can simultaneously occur due to the interlocking construction of electrode shell 46 to flex and deflect at least the distal end 78 of the electrode shell 46 to a practically unlimited number of positions. At least a portion of the electrode shell 46 can deflect in the manner described due to, for example and without limitation, impact forces on an outer surface 84 of the electrode shell 46 in use. The projections 66, 70 and recesses 68, 72 can be configured to allow the electrode shell 46 to have sufficient flexibility for deformation and/or deflection of at least a portion of the electrode shell 46 for allowing the electrode shell 46 to conform to cardiac anatomy in order to improve energy efficiency of the delivery of ablation energy. This is because the flexible electrode shell 46 can engage a larger surface area upon contact with targeted tissue 14, thereby improving contact stability and optimizing energy transfer to the targeted tissue 14 while reducing catheter induced mechanical stress.

The interlocking projections 66, 70 and recesses 68, 72 can be fabricated and/or generated by laser-cutting techniques known to those of ordinary skill in the art. For example and without limitation, electrode shell 46 is laser cut from a material suitable for surgical use, such as an electrically conductive, non-corrosive material. As described hereinabove, examples of suitable materials include gold, platinum, iridium, palladium, stainless steel, and/or any combination thereof. Projections 66, 70 and recesses 68, 72 can be laser cut out of a cylindrical piece of material. As the number of helices increases in electrode shell 46, the flexing capability of the electrode shell 46 also increases. In addition, as the pitch of the helix *(i.e.,* the distance along the axis of the helix corresponding to one turn) decreases, the ability of the electrode shell 46 to move relative to itself increases. The flexibility can be further adjusted by providing different numbers and shapes of projections 66, 70 and recesses 68, 72 to produce an electrode shell 46 that flexes to varying degrees to meet different objective. For example and without limitation, RF energy can be more specifically targeted to desired tissue areas for ablation procedures when electrode shell 46 is flexed than when it is not flexed and can provide physicians with additional positioning capability.

In accordance with another embodiment of the disclosure where the electrode shell 46 comprises a metal, the electrode shell 46 may not include interlocking projections 66, 70 and recesses 68, 72, but can instead comprise wound and/or braided metallic wires. The spacing and/or the configuration of wires, including the distance between adjacent turns of the wire can vary in accordance with various embodiments of the disclosure.

In accordance with another embodiment of the disclosure, the electrode shell 46 can comprise a polymer material. In particular, the electrode shell 46 can comprise an electrically conductive polymer. The polymer can comprise a silicone material, for example. The polymer can have electrically conductive particles dispersed therein at a predefined density in accordance with an embodiment of the disclosure. The density of the electrically conductive particles can be defined to achieve a desired electrical conductivity. The electrically conductive particles can comprise metal particles in an embodiment. For example and without limitation, the electrically conductive particles can comprise a metal such as gold, silver, platinum, iridium, titanium, tungsten, or a combination thereof. The polymer material of the electrode shell 46 can be the same as the polymer material described and illustrated in U.S. Patent Application Publication No. 2009/0171188 titled "Flexible Polymer Electrode for MRI-Guided Positioning and Radio Frequency Ablation."

Referring back to FIGS. 2-3 in particular, electrode shell 46 has a first end 78 and a second end 80. The first end 78 can be a distal end, and the second end 80 can be a proximal end in accordance with an embodiment of the disclosure. Electrode shell 46 can be generally cylindrical in shape. The first end 78 of the electrode shell 46 can be partially spherical or generally hemispherical in shape in accordance with an embodiment of the disclosure. The second end 80 of the electrode shell 46 can be configured for mechanical connection to the electrode core member 44. For example and without limitation, the second end 80 of the electrode shell 46 can be configured for mechanical connection to the first end 48 of the electrode core member 44. Electrode shell 46 can be coupled together or connected with electrode core member 44 along the same longitudinal axis 64. Electrode core member 44 and electrode shell 46 can be mechanically connected or coupled together by any known mechanisms including, for example and without limitation, adhesive bonding, press-fit configurations, snap-fit configurations, ultrasonic staking, mechanical deformation, or any other mechanism known to one of ordinary skill in the art. In an embodiment, the electrode shell 46 can be configured for mechanical connection to the first end 48 of the electrode core member 44. The first end 48 of the electrode core member 44 can have an outer diameter that is substantially equal to the inner diameter of the electrode shell 46 at the second end 80 of the electrode shell 46. The electrode core member 44 can also include a radially outwardly extending flange 82 near the first end 48 of the electrode core member 44. The radially outwardly extending flange 82 has an outer diameter that is substantially equal to the outer diameter of the proximal end 80 of the electrode shell 46.

The electrode shell 46 also has an outer surface 84 and inner surface 86 as best illustrated in FIG. 3. In an embodiment, at least one retaining wire and/or safety wire (not shown) can be extended through a lumen in the catheter shaft 18 and can be connected to the ablation electrode assembly 10. The retaining wire and/or safety wire can comprise a high tensile strength liquid crystal polymer (LCP) fiber wire in accordance with an embodiment of the disclosure. The retaining wire and/or safety wire can be configured to ensure that that the ablation electrode assembly 10 is not separated from the catheter shaft 18 to which it is attached during movement of the irrigated catheter assembly within a body 20. One end of the retaining wire and/or safety wire can be affixed in the catheter 16, for example, using an anchor pin. An opposing end of the retaining wire and/or safety wire can be affixed to the electrode shell 46. In particular, at least a portion of the retaining wire and/or safety wire can be routed through the electrode shell 46. At least a portion of the retaining wire and/or safety wire can be surface mounted to the inner surface 86 of the electrode shell 46 in accordance with an embodiment of the disclosure. At least a portion of the retaining wire and/or safety wire can be surface mounted to the inner surface 86 of the electrode shell 46 in any manner known to those of ordinary skill in the art. Referring now to FIG. 4, the electrode shell 46 can include a receptacle 87 for receiving at least a portion of the retaining wire and/or safety wire described hereinabove which can be routed through the electrode shell 46 in accordance with an embodiment of the disclosure. For example and without limitation, the electrode shell 46 can include a tab extension (not shown) extending radially inwardly from the inner surface 86 of the electrode shell 46 having at least one receptacle through which at least a portion of the retaining wire and/or safety wire can be routed and affixed to the electrode shell 46. Although a tab extension is mentioned in detail, other structures can be utilized to provide a receptacle through which the thermal sensors 58, or any number of other components, can be routed. The retaining wire and/or safety wire can be affixed to the electrode shell 46 by tying a knot in the end of the retaining wire and/or safety wire and press-fitting the knotted end into a receptacle 87. Adhesive can then be applied to bond the knot and the retaining wire and/or safety wire into the receptacle 87.

In accordance with an embodiment of the disclosure, a plug and/or bladder 88 can be configured to fill the inner volume defined by the electrode shell 46. The plug and/or bladder 88 can also provide stability for the electrode shell 46 and maintain some degree of resistance to deflection *(i.e.,* a recovery force) in some embodiments of the disclosure. The plug and/or bladder 88 is best illustrated in Fig. 3. The plug and/or bladder 88 can comprise a polymer in accordance with an embodiment of the disclosure. For example and without limitation, the polymer can comprise silicone. The plug and/or bladder 88 can be relatively soft in accordance with an embodiment of the disclosure. For example and without limitation, the durometer of the plug and/or bladder 88 can be modified and/or adjusted to provide varying degrees of flexibility based on the desired characteristics of the end user of the ablation electrode assembly 10. In other words, the plug and/or bladder 88 can have a predefined durometer to achieve a desired flexibility. The plug and/or bladder 88 can be configured to prevent ingress of blood and/or fluids into the volume defined by the electrode shell 46. The electrode shell 46 and plug and/or bladder 88 can be immediately and/or directly adjacent to each other in an embodiment of the disclosure. The electrode shell 46 and plug and/or bladder 88 can define a space therebetween in accordance with other embodiments of the disclosure. The configuration of the space can vary greatly and can be regular or irregular and can include support members (e.g., flutes, bosses, posts, and the like) to maintain separation between the electrode shell 46 and the plug and/or bladder 88 in some embodiments of the disclosure. The space can be configured as an annular space in accordance with an embodiment of the disclosure.

Electrode shell 46 can be electrically connected to an ablation system 42 to allow for the delivery of ablative energy, or the like. Electrode shell 46 can be electrically connected to an ablation system 42 in any manner conventional in the art. For example, a power wire 90 (best illustrated in FIGS. 2-3) can be provided within electrode core member 44 and electrode shell 46 of ablation electrode assembly 10. The power wire 90 can extend through a lumen(s) provided within the ablation electrode assembly 10. At least a portion of the power wire 90 can be surface mounted to the inner surface 86 of the electrode shell 46 in accordance with an embodiment of the disclosure. At least a portion of the power wire 90 can be surface mounted to the inner surface 86 of the electrode shell 46 in any manner known to those of ordinary skill in the art. Referring again to FIG. 4, the electrode shell 46 can include a receptacle 91 for receiving at least a portion of the power wire 90 described hereinabove which can be routed through the electrode shell 46 in accordance with an embodiment of the disclosure. For example and without limitation, the electrode shell 46 can include a tab extension (not shown) extending radially inwardly from the inner surface 86 of the electrode shell 46 through which at least a portion of the power wire 90 can be routed. Although a tab extension is mentioned in detail, other structures can be utilized to provide a receptacle through which the power wire 90, or any number of other components, can be routed.

Referring back to FIG. 1, the ablation system 42 can be comprised of, for example, an ablation generator 92 and one or more ablation patch electrodes 94. The ablation generator 92 generates, delivers, and controls ablation energy (e.g., RF) output by the irrigated catheter assembly and the electrode shell 46 of the ablation electrode assembly 10 thereof, in particular. The generator 92 can be conventional in the art and can comprise a commercially available unit sold under the model number IBI-1500T RF Cardiac Ablation Generator, available from St. Jude Medical, Inc. In an exemplary embodiment, the generator 92 can include an RF ablation signal source 96 configured to generate an ablation signal that is output across a pair of source connectors: a positive polarity connector SOURCE (+), which electrically connects to the electrode shell 46 of the ablation electrode assembly 10 of the irrigated catheter assembly; and a negative polarity connector SOURCE (-), can be electrically connected to one or more of the patch electrodes 94. It should be understood that the term connectors as used herein does not imply a particular type of physical interface mechanism, but is rather broadly contemplated to represent one or more electrical nodes (including multiplexed and de-multiplexed nodes). The source is configured to generate a signal at a predetermined frequency in accordance with one or more user specified control parameters (e.g., power, time, etc.) and under the control of various feedback sensing and control circuitry. The source can generate a signal, for example, with a frequency of about 450 kHz or greater for RF energy. The generator 92 can also monitor various parameters associated with the ablation procedure including, for example, impedance, the temperature at the distal tip of the irrigated catheter assembly, applied ablation energy, power, force, proximity, and the position of the irrigated catheter assembly, and provide feedback to the clinician or another component within the irrigated catheter assembly regarding these parameters.

Still referring to FIG. 1, the ablation system 42 can further include a control system 98. The control system 98 is configured to determine the temperature of the targeted tissue 14 (*i.e.,* the tissue to be ablated) and/or an appropriate ablation technique. The electrode shell 46 of the ablation electrode assembly 10 can be connected to the control system 98 with wires. The ablation generator 92 can form part of the control system 98 in accordance with some embodiments or can be separate from the control system 98 in other embodiments. The thermal sensors 58 can be connected to the control system 98. For example and without limitation, wires can extend through lumens in the catheter. Devices for determining pressure, temperature, and a flow parameter of a flowing fluid available from Radi Medical Systems AB, and as generally shown with reference to at least U.S. Patent No. RE39,863 entitled "Combined flow, pressure and temperature sensor," can be used to monitor and/or control the quantity of flow of irrigation fluid within or from the catheter at one or more locations using a flow-from pressure algorithm as described therein or as known to those of ordinary skill in the art. These devices for determining pressure, temperature, and a flow parameter of a flowing fluid can also be connected to the control system 98. The energy provided to the ablation electrode assembly 10 can be increased by the control system 98 by increasing the power and/or length of energy delivery (e.g., amplitude and/or operating time) during the ablation cycle. The energy provided to the ablation electrode assembly 10 can be decreased by decreasing the power and/or length of time of energy delivery (e.g., frequency and/or operating time) during the ablation cycle. The ablation technique that is selected by the control system 98 can be selected to produce a certain, predetermined temperature in the targeted tissue 14 that will form a desired lesion in the targeted tissue 14. While the desired lesion can be transmural in some embodiments, the characteristics of the desired lesion can vary significantly. The certain, predetermined temperature in the targeted tissue 14 that will form a desired lesion in the targeted tissue 14 can be affected by the thermal response of the targeted tissue. The thermal response of the targeted tissue 14 can be affected by a number of variables including tissue thickness, amount of fat and muscle, blood flow through the region, and blood flow at the interface of the ablation electrode assembly 10 and the targeted tissue 14.

Referring back to FIGS. 2-4, in accordance with an embodiment of the disclosure, the ablation electrode assembly 10 further includes an irrigant distribution element 100. Irrigant distribution element 100 can be configured as a generally annular ring in accordance with an embodiment of the disclosure. The irrigant distribution element 100 has a first end 102 and a second end 104. The first end 102 can be a proximal end, and the second end 104 can be a distal end in accordance with an embodiment of the disclosure. At least a portion of the first end 102 of the irrigant distribution element 100 can engage a catheter shaft 18 in which the electrode core member 44 can be located. At least a portion of the second end 104 of the irrigant distribution element 100 can surround and/or encircle at least a portion of the electrode core member 44 and further, can define a circumferential irrigation port 106 between the irrigant distribution element 100 and the electrode core member 44 in accordance with an embodiment of the disclosure.

Irrigant distribution element 100 is configured to guide irrigation fluid toward electrode shell 46 about and along outer surface 84 of the electrode shell 46, and in particular, direct the fluid (e.g., irrigant) flow in a direction substantially parallel with the outer surface 84 of the electrode shell 46. Irrigant distribution element 100 can include a fluid shaping member 108 that helps ensure that the fluid flow tends toward the surface 84 of the electrode shell 46 of the ablation electrode assembly 10. For example and without limitation, the fluid shaping member 108 of the irrigant distribution element 100 can include a channel, rifling, boss, hump, chamfer, and/or combination thereof on a surface of the irrigant distribution element 100 defining the circumferential irrigation port 106. The fluid shaping member 108 is configured to disturb fluid flow (e.g., cause fluid flowing closer to the outer surface 52 of the electrode core member 44 to slow down relative to fluid flowing farther from the outer surface 52 of the electrode core member 44), thereby helping to ensure that the fluid flow tends toward the surface 84 of the electrode shell 46. In this way, the flow of irrigant can be turbulent in order to provide an enveloping flow pattern adjacent to the outer surface 84 of the electrode shell 46 of the ablation electrode assembly 10 for mixing with, displacing, and/or diluting blood that can be in contact with the ablation electrode assembly 10 in order to help prevent stasis and the formation of coagulum. Although flexing of the electrode shell 46 can affect the flow of irrigant, it is expected that the flexing of the electrode shell 46 will not have a significant clinical impact since any flexing and/or deflection of the electrode shell 46 is limited and relatively small in accordance with an embodiment of the disclosure.

The configuration of irrigant distribution element 100 can improve fluid flow of the irrigation fluid such that the total flow rate (or volume delivered per unit of time) of irrigation fluid can be exceedingly low as compared to traditional irrigation flow rates (and volumes). In other words, overall total fluid volumes of irrigation fluid can be much lower than the prior art or than those fluid volumes typically utilized in clinical practice, which can be especially valuable for patients already suffering from fluid overload (e.g., patient having heart failure and the like). Overall total fluid volume can range from low single digits to about ten or so milliliters per minute while effectively reducing or eliminating char and coagulum and improving temperature correlation for precise control of power to maintain a temperature during ablation procedures.

Valve members, for example and without limitation, such as those shown and described in co-owned U.S. Patent Application Publication No. 2008/0161795 entitled "Irrigated Ablation Catheter System With Pulsatile Flow To Prevent Thrombus," or other similar flow control features can be used in connection with catheters incorporating ablation electrode assembly 10 in order to change the flow rate of irrigation fluid. In other embodiments, the flow control features can be part of an ancillary control system separate from and to be used in conjunction with catheters. The valves can operate automatically without user input and/or can operate based on feedback recorded during RF ablation by the ECU of the visualization, navigation, and/or mapping system 30. The feedback can relate to time, temperature, and/or impedance, for example and without limitation. Circuitry for implementing the feedback automatically in a control algorithm can be readily provided by those having ordinary skill in the art after becoming familiar with the teachings herein.

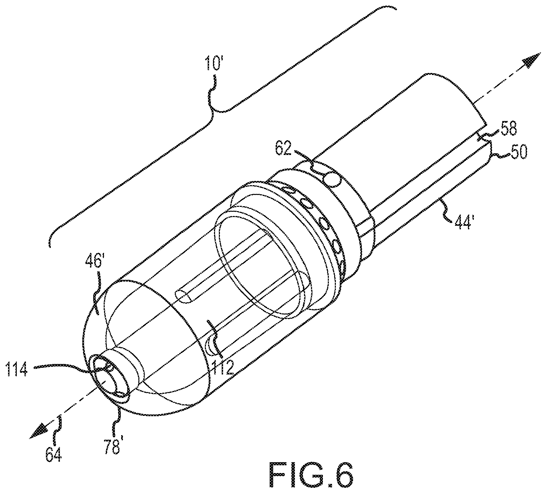

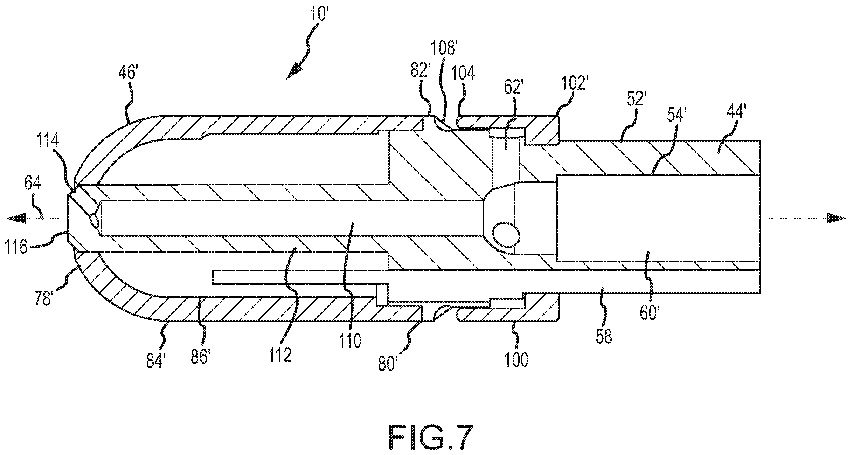

Referring now to FIGS. 6-7, ablation electrode assembly 10' can include an electrode core member 44' and an electrode shell 46' in accordance with a second embodiment of the disclosure and in accordance with an embodiment of the present invention. The ablation electrode assembly 10' in accordance with a second embodiment of the disclosure can be substantially identical to the ablation electrode assembly 10 as described hereinabove including the electrode shell 46' being generally flexible in an embodiment (e.g., configured to conform to the targeted tissue 14 by deflection and/or deformation when the electrode shell 46' comes into physical contact with the targeted tissue 14), except that the electrode core member 44' and/or the electrode shell 46' can be modified to provide distal delivery of irrigation fluid in which at least a portion of the irrigation fluid is transferred to a distal exhaust port. The ablation electrode assembly 10' that is configured to provide both proximal and distal delivery of irrigation fluid can be especially beneficial to reduce thrombus formation and/or charring at the distal end (*e.g.,* tip) of the ablation electrode assembly 10'. By providing both proximal and distal delivery of irrigation fluid, it can further displace blood and prevent stasis in the areas adjacent the electrode shell 46' of the ablation electrode assembly 10'.

Still referring to FIGS. 6-7, ablation electrode assembly 10' is configured for distal delivery of irrigation fluid with an axially extending passageway 110 extending from the inner cavity 60' of the electrode core member 44' toward the first end 78' of the electrode shell 46'. The axially extending passageway 110 can be defined by a generally cylindrical member 112. The generally cylindrical member 112 can be integral with the inner core member 44' in accordance with various embodiments of the disclosure. The generally cylindrical member 112 can also be separate from the inner core member 44' in accordance with various other embodiments of the disclosure. For example and without limitation, the generally cylindrical member 112 may comprise a close wound coil spring with a liner or jacket of low durometer polymer. For another example, the generally cylindrical member 112 may comprise a polymer tube. The cylindrical member 112 can be sufficiently flexible so that at least a portion of the cylindrical member 112 is configured for deformation and/or deflection in a number of directions relative to the longitudinal axis 64 of ablation electrode assembly 10. Although the member 112 is defined as generally cylindrical, the member 112 can comprise any number of various shapes in accordance with embodiments of the disclosure. In accordance with another embodiment of the disclosure, the axially extending passageway 110 can be defined by a through-hole disposed in the plug and/or bladder 88 configured to fill the inner volume defined by the electrode shell 46. As described hereinabove, the plug and/or bladder 88 can comprise silicone in accordance with an embodiment of the invention. The total range of deflection of cylindrical member 112 and/or the plug and/or bladder 88 can be relatively small such that stress on the conduit is not expected to adversely affect the function of the ablation electrode assembly 10'.

The axially extending passageway 110 extends to the distal end 78' of the electrode shell 46'. As generally illustrated in FIGS. 6-7, the axially extending passageway 110 transitions into one or more ports 114 near the distal end 116 of the member 112. Port(s) 114 enable irrigation fluid flowing through the axially extending passageway 110 to flow to a first end 78' of the electrode shell 46', therein substantially irrigating the first end 78' (*e.g.,* tip) of electrode shell 46' of the ablation electrode assembly 10 '. For example and without limitation, the member 112 can include three ports 114. Each of the port(s) 114 is oriented at a generally acute angle (e.g., about 45 degrees) relative to the longitudinal axis 64 of the ablation electrode assembly 10'. The orientation of the port(s) 114 varies depending on the design of the ablation electrode assembly 10'. The port(s) 114 can be substantially equally spaced around the circumference of the member 112 in an embodiment of the disclosure. The port(s) 114 are configured to extend from the distal end of the axially extending passageway 110 to the distal end 78' of the electrode shell 46'.

In an embodiment of the disclosure, a coating (not shown) can be disposed on at least a portion of the member 112 that defines the axially extending passageway 100. For example and without limitation, a coating can be especially useful if the member 112 is not integral with the inner core member 44' and instead comprises a material that may be electrically conductive. The coating can be comprised of an electrically non-conductive material. For example and without limitation, the coating can be comprised of diamond, diamond-like carbon (DLC), or polytetrafluoroethylene (PTFE), which is commonly sold by the E. I. du Pont de Nemours and Company under the trademark TEFLON®. In an embodiment of the disclosure, the coating can be provided around the entire circumference and along the entire length of the axially extending passageway 110. However, the coating can be provided only around a portion of the circumference and/or only along a portion of the length of the axially extending passageway 110 in accordance with various embodiments of the disclosure. The amount of the coating provided around the circumference and/or length of the axially extending passageway 110 or portion thereof can vary depending on the relative requirements of ablation electrode assembly 10'.

Although ablation electrode assemblies 10, 10' are described and illustrated with a single electrode core member 44, 44' and a single electrode shell 46, 46', an ablation catheter 16 can include two or more electrode core members 44, 44' and/or two or more electrode shells 46, 46' in accordance with various embodiments of the disclosure. Furthermore, although ablation electrode assemblies 10, 10' are described and illustrated such that the electrode shell 46, 46' is located distally of the electrode core member 44, 44', at least one electrode shell 46, 46' can be located proximally of an electrode core member 44, 44' in accordance with various embodiments of the disclosure.

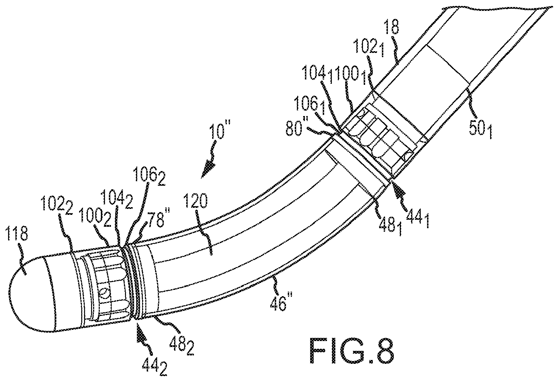

For example and without limitation, an ablation electrode assembly 10" can include two or more electrode core members 44₁, 44₂ and a single electrode shell 46" as generally illustrated in FIG. 8. The first electrode core member 44₁ can be disposed proximally relative to the electrode shell 46. The first electrode core member 44₁ can be substantially identical to the electrode core member 44, 44' described hereinabove. The second electrode core member 44₂ can be disposed distally relative to the electrode shell 46. The second electrode core member 44₂ can be substantially identical to the electrode core member 44, 44' described hereinabove; however, the second electrode core member 44₂ can be oriented such that the first and second electrode core members 44₁, 44₂ face in opposing directions. Accordingly, the first end 48₂ of the second electrode core member 44₂ can be a proximal end, and the second end (not shown) of the second electrode core member 44₂ can be a distal end. Electrode shell 46" can be substantially identical to the electrode shell 46 described herein above. Electrode shell 46" can be generally cylindrical in shape, and both the first and second ends 78" , 80" of the electrode shell 46" can be open. In particular, the first end 78" of the electrode shell 46" can be configured for connection to the second electrode core member 44₂ that is located distally of the electrode shell 46", and the second end 78" of the electrode shell 46" can be configured for connection to the first electrode core member 44₁ that is located proximally of the electrode shell 46".

The ablation electrode assembly 10" generally illustrated in FIG. 8 can also include two or more irrigant distribution elements 100₁, 100₂. Irrigant distribution elements 100₁, 100₂ can be substantially identical to the irrigant distribution element 100 described hereinabove. At least a portion of the first end 102₁ of the first irrigant distribution element 100₁ can engage a catheter shaft 18 in which the first electrode core member 44₁ can be located. At least a portion of the second end 104₁ of the first irrigant distribution element 100₁ can surround and/or encircle the first electrode core member 44₁ and further, can define a circumferential irrigation port 106₁ between the first irrigant distribution element 100₁ and the electrode core member 44₁. The circumferential irrigation port 106₁ is configured to guide irrigation fluid toward electrode shell 46", and therefore, directs the irrigation fluid distally. At least a portion of the first end 102₂ of the second irrigant distribution element 100₂ can engage a tip electrode 118. The tip electrode 118 may or may not be flexible in accordance with various embodiments of the disclosure. At least a portion of the second end 104₂ of the second irrigant distribution element 100₂ can surround and/or encircle the second electrode core member 44₂, and further, can define a circumferential irrigation port 106₂ between the second irrigant distribution element 100₂ and the second electrode core member 44₂. The circumferential irrigation port 106₂ is configured to guide irrigation fluid toward electrode shell 46", and therefore, directs the irrigation fluid proximally. An irrigant supply line 120 can be disposed between the first electrode core member 44₁ and the second electrode core member 44₂. The irrigant supply line 120 can be the same as or can be in fluid communication with the fluid delivery tube 22 disposed within the catheter shaft 18.

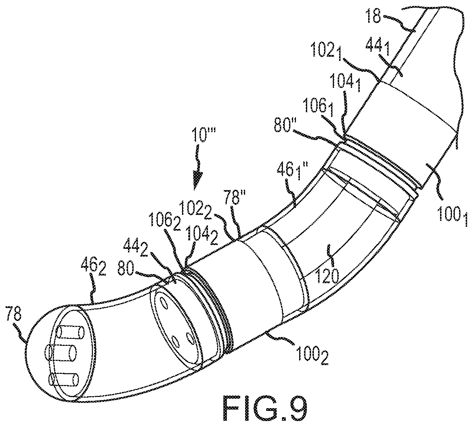

Referring now to FIG. 9, ablation electrode assembly 10''' can include two ore more electrode core members 44₁, 44₂ and two or more electrode shells 46"₁, 46₂. Although two electrode core members 44₁, 44₂ and two electrode shells 46"₁, 46₂ are generally illustrated, the ablation electrode assembly 10''' can include any number of electrode core members 44 and electrode shells 46 in accordance with various embodiments of the disclosure. The first electrode core member 44₁ can be disposed proximally relative to the first electrode shell 46"₁. The first electrode core member 44₁ can be substantially identical to the electrode core member 44, 44' described hereinabove. In addition, the first electrode shell 46"₁ can be substantially identical to the electrode shell 46 described hereinabove. The first electrode shell 46"₁ can be generally cylindrical in shape, and both the first and second ends 78" , 80" of the first electrode shell 46" ₁ can be open. In particular, the first end 78" of the first electrode shell 46"₁ can be configured for connection to the second electrode core member 44₂ that is located distally of the first electrode shell 46"₁, and the second end 80" of the electrode shell 46 "₁ can be configured for connection to the first electrode core member 44₁ that is located proximally of the first electrode shell 46".

The second electrode core member 44₂ can be disposed proximally relative to the second electrode shell 46₂. The second electrode core member 44₂ can be substantially identical to the electrode core member 44, 44' described hereinabove. The second electrode shell 46₂ can also be generally cylindrical in shape. First end 78 of the second electrode shell 46₂ can be closed and can be hemispherical and/or spherical in shape. The first end 78 of the second electrode shell 46₂ can be hemispherical and/or spherical in shape when the second electrode shell 46₂ is disposed at the distal tip of the ablation electrode assembly 10'''. However, the second electrode shell 46₂ does not have to be disposed at the distal tip of the ablation electrode assembly. Accordingly, in other embodiments of the disclosure, the second electrode shell 46₂ can be disposed at any location along the ablation catheter 16. Depending upon the location of the second electrode shell 46₂, the first end 78 of the second electrode shell 46₂ can be open or closed. The second end 80 of the second electrode shell 462 can be open and can be configured for connection to the second electrode core member 44₂.

In some embodiments, the ablation electrode assembly 10'" generally illustrated in FIG. 9 can include two or more irrigant distribution elements 100₁, 100₂. Irrigant distribution elements 100₁, 100₂ can be substantially identical to the irrigant distribution element 100 described hereinabove. At least a portion of the second end 104₁, 104₂ of each irrigant distribution element 100₁, 100₂ can surround and/or encircle each corresponding electrode core member 44₁, 44₂, and further can define a circumferential irrigation port 106₁, 106₂ between the irrigant distribution element 100₁, 100₂ and the electrode core member 44₁, 44₂. Each circumferential irrigation port 106₁, 106₂ is configured to guide irrigation fluid toward electrode shell 46"₁, 46₂, and therefore, each irigant distribution elements 100₁, 100₂ directs the irrigation fluid distally. An irrigant supply line 120 can be disposed between the first electrode core member 44₁ and the second electrode core member 44₂. The irrigant supply line 120 can be in fluid communication with the fluid delivery tube 22 disposed within the catheter shaft 18. In accordance with various embodiments of the invention (and as generally illustrated in FIGS. 8-9), ablation electrode assemblies can include a series of two or more active ablation electrodes each with its own dependent (*e.g.,* common source) or independent (*e.g.,* discrete source) irrigant distribution configuration.

Although at least four embodiments of this disclosure have been described above with a certain degree of particularity, those skilled in the art could make numerous alterations to the disclosed embodiments without departing from the scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and can include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting Changes in detail or structure can be made without departing from the disclosure as defined in the appended claims.

## Claims

1. An ablation electrode assembly (10') having a longitudinal axis, the assembly comprising:
an electrode core member (44') comprising a thermal insulator having a reduced thermal conductivity, the electrode core member (44') having:
a distal end (48);
a proximal end (50); and
at least one irrigation passageway (62');
an electrode shell (46') comprising an electrically conductive material, the electrode shell (46') defining an inner volume and the electrode shell (46') having:
a distal end (78'); and
a proximal end (80'), wherein the proximal end (80') of the electrode shell (46') is configured for connection to the distal end (48) of the electrode core member (44') and wherein the electrode shell (46') is sufficiently flexible for deflection of the distal end (78') of the electrode shell (46') relative to the longitudinal axis of the ablation electrode assembly (10');
an irrigant distribution element (100) surrounding at least a portion of the electrode core member (44'), the irrigant distribution element (100) having:
a proximal end (102'); and
a distal end (104), wherein the distal end (104) of the irrigant distribution element (100) defines a circumferential irrigation port (106) between the irrigant distribution element (100) and the electrode core member (44'), the irrigation port (106) being fluidly connected to the at least one irrigation passageway (62'),
wherein the electrode core member (44') further comprises:
an outer surface (52');
an inner surface (54') defining an inner cavity (60'), wherein the at least one irrigation passageway (62') extends from the inner cavity (60') to the outer surface (52') of the electrode core member (44'), and
an axially extending passageway (110) extending from the inner cavity (60') of the electrode core member (44') toward the distal end (78') of the electrode shell (46'),
further comprising at least one port (114) extending from the axially extending passageway (110) to the distal end (78') of the electrode shell (46'), wherein the port (114) is oriented at an acute angle relative to the longitudinal axis of the ablation electrode assembly (10').

2. The ablation electrode assembly (10') of claim 1, wherein at least a portion of the circumference and at least a portion of the length of the axially extending passageway (110) includes a coating of an electrically non-conductive material.

3. The ablation electrode assembly (10') of claim 1, wherein at least a portion of the electrode shell (46') includes a first set of projections (66) defining at least in part a corresponding first set of recesses (68) and wherein at least a portion of the electrode shell (46') includes a second set of projections (70) defining at least in part a corresponding second set of recesses (72), wherein at least one of the first set of projections (66) is configured to interlock with at least one of the second set of recesses (72), and wherein at least one of the second set of projections (70) is configured to interlock with at least one of the first set of recesses (68).

4. The ablation electrode assembly (10') of claim 1, wherein the electrode shell (46') comprises a polymer.

5. The ablation electrode assembly (10') of claim 4, wherein the polymer has electrically conductive particles dispersed therein at a predefined density to achieve a desired electrical conductivity.

6. The ablation electrode assembly (10') of claim 1, wherein the irrigant distribution element (100) is an annular ring.

7. The ablation electrode assembly (10') of claim 1, wherein the irrigant distribution element (100) further comprises a fluid shaping member (108'), wherein the fluid shaping member (108') comprises at least one of a channel, rifling, boss, hump, and chamfer, or combination thereof.

## Patentansprüche

1. Ablationselektrodenbaugruppe (10') mit einer Längsachse, wobei die Baugruppe enthält:
ein Elektrodenkernelement (44'), das einen thermischen Isolierkörper aufweist, der eine reduzierte thermische Leitfähigkeit aufweist, wobei das Elektrodenkernelement (44') aufweist:
ein entferntes Ende (48);
ein nahes Ende (50); und
wenigstens einen Spüldurchgang (62');
eine Elektrodenhülle (46'), die ein elektrisch leitendes Material enthält, wobei die Elektrodenhülle (46') ein Innenvolumen definiert und die Elektrodenhülle (46') aufweist:
ein entferntes Ende (78'); und
ein nahes Ende (80'), wobei das nahe Ende (80') der Elektrodenhülle (46') ausgebildet ist zum Verbinden mit dem entfernten Ende (48) des Elektrodenkernelements (44') und wobei die Elektrodenhülle (46') ausreichend elastisch ist für eine Biegung des entfernten Endes (78') der Elektrodenhülle (46') relativ zu der Längsachse der Ablationselektrodenbaugruppe (10');
ein Spülmittel-Verteilelement (100), das wenigstens einen Bereich des Elektrodenkernelements (44') umgibt, wobei das Spülmittel-Verteilelement (100) aufweist:
ein nahes Ende (102'); und
ein entferntes Ende (104), wobei das entfernte Ende (104) des Spülmittel-Verteilelements (100) eine Umfangsspülöffnung (106) zwischen dem Spülmittel-Verteilelement (100) und dem Elektrodenkernelement (44') definiert, wobei die Spülöffnung (106) fluidmäßig mit dem wenigstens einen Spüldurchgang (62') verbunden ist,
wobei das Elektrodenkernelement (44') ferner aufweist:
eine Außenfläche (52');
eine Innenfläche (54'), die eine Innenaussparung (60') definiert, wobei der wenigstens eine Spüldurchgang (62') sich von der Innenaussparung (60') zu der Außenfläche (52') des Elektrodenkernelements (44') erstreckt, und
ein sich axial erstreckender Durchgang (110), der sich von der Innenaussparung (60') des Elektrodenkernelements (44') in Richtung zu dem entfernten Ende (78') der Elektrodenhülle (46') erstreckt,
ferner enthaltend wenigstens eine Öffnung (114), die sich von dem sich axial erstreckenden Durchgang (110) zu dem entfernten Ende (78') der Elektrodenhülle (46') erstreckt, wobei die Öffnung (114) in einem spitzen Winkel relativ zu der Längsachse der Ablationselektrodenbaugruppe (10') ausgerichtet ist.

2. Ablationselektrodenbaugruppe (10) nach Anspruch 1, wobei wenigstens ein Bereich des Umfangs und ein Bereich der Länge des sich axial erstreckenden Durchgangs (110) eine Beschichtung aus einem elektrisch nicht leitenden Material aufweist.

3. Ablationselektrodenbaugruppe (10') nach Anspruch 1, wobei wenigstens ein Bereich der Elektrodenhülle (46') einen ersten Satz Vorsprünge (66) aufweist, der wenigstens zum Teil einen entsprechenden ersten Satz Aussparungen (68) definiert, und wobei wenigstens ein Bereich der Elektrodenhülle (46') einen zweiten Satz Vorsprünge (70) aufweist, der wenigstens zum Teil einen entsprechenden zweiten Satz von Aussparungen (72) definiert, wobei wenigstens einer aus dem ersten Satz von Vorsprüngen (66) ausgebildet ist zum Verzahnen mit wenigstens einem aus dem zweiten Satz von Aussparungen (72), und wobei wenigstens einer aus dem zweiten Satz von Vorsprüngen (70) ausgebildet ist zum Verzahnen mit wenigstens einem aus dem ersten Satz von Aussparungen (68).

4. Ablationselektrodenbaugruppe (10') nach Anspruch 1, wobei die Elektrodenhülle (46') ein Polymer aufweist.

5. Ablationselektrodenbaugruppe (10') nach Anspruch 4, wobei das Polymer elektrisch leitende Partikel aufweist, die darin in einer vordefinierten Dichte verteilt sind zum Erzielen einer gewünschten elektrischen Leitfähigkeit.

6. Ablationselektrodenbaugruppe (10') nach Anspruch 1, wobei das Spülmittel-Verteilelement (100) ein ringförmiger Ring ist.

7. Ablationselektrodenbaugruppe (10') nach Anspruch 1, wobei das Spülmittel-Verteilelement (100) ferner ein Fluidformungselement (108') aufweist, wobei das Fluidformungselement (108') wenigstens eines aus einem Kanal, einem Drall, einem Vorsprung, einem Buckel und einer Fase oder eine Kombination davon aufweist.

## Revendications

1. Ensemble d'électrodes d'ablation (10') présentant un axe longitudinal, l'ensemble comprenant :
un élément de noyau d'électrode (44') comprenant un isolant thermique présentant une conductivité thermique réduite, l'élément de noyau d'électrode (44') présentant :
une extrémité distale (48) ;
une extrémité proximale (50) ; et
au moins un passage d'irrigation (62') ;
une gaine d'électrode (46') comprenant un matériau électriquement conducteur, la gaine d'électrode (46') définissant un volume intérieur et la gaine d'électrode (46') présentant :
une extrémité distale (78') ; et
une extrémité proximale (80'), dans lequel l'extrémité proximale (80') de la gaine d'électrode (46') est configurée pour être reliée à l'extrémité distale (48) de l'élément de noyau d'électrode (44') et dans lequel la gaine d'électrode (46') est suffisamment souple pour subir une déflexion de l'extrémité distale (78') de la gaine d'électrode (46') par rapport à l'axe longitudinal de l'ensemble d'électrodes d'ablation (10') ;
un élément de distribution d'irrigation (100) entourant au moins une partie de l'élément de noyau d'électrode (44'), l'élément de distribution d'irrigation (100) présentant :
une extrémité proximale (102') ; et
une extrémité distale (104), dans lequel l'extrémité distale (104) de l'élément de distribution d'irrigation (100) définit un orifice d'irrigation circonférentiel (106) entre l'élément de distribution d'irrigation (100) et l'élément de noyau d'électrode (44'), l'orifice d'irrigation (106) étant relié de manière fluidique audit au moins un passage d'irrigation (62'),
dans lequel l'élément de noyau d'électrode (44') comprend en outre :
une surface extérieure (52') ;
une surface intérieure (54') définissant une cavité intérieure (60'), dans lequel ledit au moins un passage d'irrigation (62') s'étend de la cavité intérieure (60') à la surface extérieure (52') de l'élément de noyau d'électrode (44'), et
un passage (110) s'étendant axialement depuis la cavité intérieure (60') de l'élément de noyau d'électrode (44') vers l'extrémité distale (78') de la gaine d'électrode (46'),
comprenant en outre au moins un orifice (114) s'étendant du passage (110) s'étendant axialement à l'extrémité distale (78') de la gaine d'électrode (46'), dans lequel l'orifice (114) est orienté selon un angle aigu par rapport à l'axe longitudinal de l'ensemble d'électrodes d'ablation (10').

2. Ensemble d'électrodes d'ablation (10') selon la revendication 1, dans lequel au moins une partie de la circonférence et au moins une partie de la longueur du passage (110) s'étendant axialement comprend un revêtement d'un matériau électriquement non conducteur.

3. Ensemble d'électrodes d'ablation (10') selon la revendication 1, dans lequel au moins une partie de la gaine d'électrode (46') comprend un premier ensemble de projections (66) définissant au moins en partie un premier ensemble correspondant d'évidements (68) et dans lequel au moins une partie de la gaine d'électrode (46') comprend un deuxième ensemble de projections (70) définissant au moins en partie un deuxième ensemble correspondant d'évidements (72), dans lequel au moins une projection parmi le premier ensemble de projections (66) est configurée pour se verrouiller avec au moins un évidement parmi le deuxième ensemble d'évidements (72), et dans lequel au moins une projection parmi le deuxième ensemble de projections (70) est configurée pour se verrouiller avec au moins un évidement parmi le premier ensemble d'évidements (68).

4. Ensemble d'électrodes d'ablation (10') selon la revendication 1, dans lequel la gaine d'électrode (46') comprend un polymère.

5. Ensemble d'électrodes d'ablation (10') selon la revendication 4, dans lequel le polymère contient des particules électriquement conductrices dispersées à une densité prédéfinie pour obtenir une conductivité électrique souhaitée.

6. Ensemble d'électrodes d'ablation (10') selon la revendication 1, dans lequel l'élément de distribution d'irrigation (100) est une bague annulaire.

7. Ensemble d'électrodes d'ablation (10') selon la revendication 1, dans lequel l'élément de distribution d'irrigation (100) comprend en outre un élément de mise en forme de fluide (108'), dans lequel l'élément de mise en forme de fluide (108') comprend au moins un élément parmi un canal, une rayure, un bossage, une bosse et un chanfrein, ou une combinaison de ceux-ci.
